# EUROPÄISCHE PATENTSCHRIFT

(11) **EP 2 143 392 B1**
(45) Veröffentlichungstag und Bekanntmachung des Hinweises auf die Patenterteilung: **16.12.2015**
(21) Anmeldenummer: 09163064.0
(22) Anmeldetag: 18.06.2009
(51) Int. Cl.: A61B 17/34

(54) **Chirurgische Dichtelementhalterung zum Halten eines chirurgischen Dichtelements und chirurgisches Abdichtungssystem**
Surgical seal element holder for holding a surgical seal element and surgical sealing system
Fixation d'élément étanche chirurgical pour maintenir un élément étanche chirurgical et système d'étanchéification chirurgical

(30) Priorität: 09.07.2008 DE 102008033375
(43) Veröffentlichungstag der Anmeldung: 13.01.2010
(73) Patentinhaber: Aesculap AG, 78532 Tuttlingen (DE)
(72) Erfinder: Mayenberger, Rupert, 78239, Rielasingen (DE); Schweitzer, Tom, 78532, Tuttlingen (DE)
(74) Vertreter: Hoeger, Stellrecht & Partner Patentanwälte mbB

(56) Entgegenhaltungen:
- EP-A1- 1 716 813
- WO-A2-02/41795
- WO-A2-2004/096295
- US-A1- 2004 260 244

## Beschreibung

Die vorliegende Erfindung betrifft eine chirurgische Dichtelementhalterung zum Halten eines chirurgischen, eine erweiterbare Einführöffnung aufweisenden Dichtelements eines chirurgischen, einen Trokar mit einer Trokarhülse umfassenden Abdichtungssystems, wobei die Dichtelementhalterung ein Halterungsdichtelement zum Abdichten der Dichtelementhalterung mit Bezug zu einer inneren Wandfläche der Trokarhülse umfasst und wobei das Halterungsdichtelement in Form eines in radialer Richtung von der Dichtelementhalterung abstehenden Flansches ausgebildet ist.

Ferner betrifft die vorliegende Erfindung ein chirurgisches Abdichtungssystem umfassend einen Trokar mit einer Trokarhülse und eine chirurgische Dichtelementhalterung zum Halten eines chirurgischen, eine erweiterbare Einführöffnung aufweisenden Dichtelements, wobei die Dichtelementhalterung ein Halterungsdichtelement zum Abdichten der Dichtelementhalterung mit Bezug zu einer inneren Wandfläche der Trokarhülse aufweist.

Eine chirurgische Dichtelementhalterung zum Halten eines chirurgischen, eine erweiterbare Einführöffnung aufweisenden Dichtelements eines chirurgischen, einen Trokar mit einer Trokarhülse umfassenden Abdichtungssystems sowie ein chirurgisches Abdichtungssystem umfassend einen Trokar mit einer Trokarhülse und eine chirurgische Dichtelementhalterung zum Halten eines chirurgischen, eine erweiterbare Einführöffnung aufweisenden Dichtelements sind beispielsweise aus der DE 20 2006 005 442 U1 bekannt. Derartige Abdichtungssysteme sind üblicherweise ganz oder teilweise wiederverwendbar ausgestaltet. Insbesondere bei Dichteinheiten der Abdichtungssysteme kann es zu einem erhöhten Verschleiß aufgrund des Einführens von Instrumenten kommen, so dass diese nur eine begrenzte Zahl von Wiederaufbereitungszyklen, das heißt insbesondere Reinigung und nachfolgende Sterilisation, durchlaufen können. Insbesondere bei nur teilweise wiederverwendbaren Systemen ist es wichtig, dass das Dichtelement auf einfache und sichere Weise ausgetauscht und ein von der Trokarhülse definierter Kanal sicher gegen Gasverlust abgedichtet werden kann.

Weitere Dichtelementhalterungen und Abdichtungssysteme sind aus der EP 1 716 813 A1, der US 2004/0260244 A1, der WO 02/41795 A2 sowie der WO 2004/096295 A2 bekannt. EP 1716813 A1 stellt den nächsten Stand der Technik dar.

Es ist daher Aufgabe der vorliegenden Erfindung, eine chirurgische Dichtelementhalterung und ein chirurgisches Abdichtungssystem der eingangs beschriebenen Art so zu verbessern, dass eine jederzeit perfekte Abdichtung gegenüber einem Kanal der Trokarhülse gewährleistet ist.

Diese Aufgabe wird bei einer chirurgischen Dichtelementhalterung sowie einem chirurgischen Abdichtungssystem der eingangs beschriebenen Art erfindungsgemäß dadurch gelöst, dass der Flansch etwas in distaler Richtung weisend bezogen auf eine quer zu einer Längsachse der Dichtelementhalterung verlaufende Ebene geneigt ist.

Durch das Halterungsdichtelement können auf einfache Weise Fluidverluste, insbesondere Gasverluste, durch das Abdichtungssystem hindurch verhindert werden. Ein durch die Trokarhülse definierter Instrumenteneinführkanal kann so optimal abgedichtet werden, zum einen durch das Halterungsdichtelement und zum anderen insbesondere durch ein in die Dichtelementhalterung eingesetztes Dichtelement. Die Dichtelementhalterung hat ferner den Vorteil, dass insbesondere Verschleißteile des Dichtungssystems, beispielsweise das Dichtelement, auf einfache Weise ausgetauscht werden können. Das Halterungsdichtelement kann zudem eine spielfreie Fixierung der Dichteinheit in der Trokarhülse oder einem Dichtungsgehäuse derselben sicherstellen. Des Weiteren lassen sich Fertigungstoleranzen der Dichtelementhalterung und/oder der Trokarhülse durch das Halterungsdichtelement einfach und sicher ausgleichen. Es werden keine weiteren Dichtungselemente benötigt, um die Dichtelementhalterung relativ zur Trokarhülse abzudichten. Dies vereinfacht sowohl die Montage als auch die Demontage der Dichtelementhalterung von der Trokarhülse. Des Weiteren können so auch Kosten des Abdichtungssystems insgesamt reduziert werden, da beispielsweise das Halterungsdichtelement direkt in die Dichtelementhalterung integriert werden kann. Besonders einfach in der Konstruktion sowie in der Herstellung wird die Dichtelementhalterung dadurch, dass das Halterungsdichtelement in Form eines in radialer Richtung von der Dichtelementhalterung abstehenden Flansches ausgebildet ist. Das Halterungsdichtelement lässt sich so direkt in die Dichtelementhalterung integrieren. Besonders gut lassen sich Fertigungstoleranzen durch die Dichtelementhalterung dadurch ausgleichen, dass der Flansch etwas in distaler Richtung weisend bezogen auf eine quer zu einer Längsachse der Dichtelementhalterung verlaufende Ebene geneigt ist. Ein Neigungswinkel kann in einem Bereich von 1° bis 15° liegen, vorzugsweise in einem Bereich von 1° bis 8°. Zum Abdichten kann so der Flansch nach Anlegen an eine entsprechende Dichtfläche der Trokarhülse etwas in proximaler Richtung verformt werden und kann so insbesondere unter Vorspannung gegen die Dichtfläche drücken, um eine dauerhafte Abdichtung sicherzustellen. Auch der Ausgleich von Fertigungstoleranzen an der Trokarhülse und/oder der Dichtelementhalterung kann so auf einfache Weise erreicht werden.

Günstigerweise ist die Dichtelementhalterung ausgebildet zum lösbaren Verbinden mit der Trokarhülse. Dies gestattet es, die Dichtelementhalterung mit einem daran angeordneten Dichtelement, schnell, einfach und sicher auszutauschen.

Besonders einfach und kostengünstig herstellen lässt sich die Dichtelementhalterung, wenn das Halterungsdichtelement einstückig mit der Dichtelementhalterung ausgebildet ist. Die Dichtelementhalterung kann so beispielsweise aus einem Kunststoff durch Spritzgießen in einem Arbeitsschritt hergestellt werden.

Vorteilhafterweise ist das Halterungsdichtelement mindestens abschnittsweise elastisch verformbar. Insbesondere können so Fertigungstoleranzen auf einfache Weise ausgeglichen werden. Günstig ist auch eine flexible, axial etwas federnde Ausgestaltung des Halterungsdichtelements. Unter einer abschnittsweisen elastischen Verformbarkeit ist insbesondere eine Verformbarkeit längs eines Teil einer radialen Erstreckung des Halterungsdichtelements zu verstehen.

Günstig ist es, wenn das Halterungsdichtelement eine weitere Dichtung trägt. Insbesondere dann, wenn das Halterungsdichtelement selbst nur wenig oder gar nicht elastisch oder flexibel ausgebildet ist, ermöglichst es die weitere Dichtung, eine optimale Abdichtung relativ zur Trokarhülse zu realisieren. Vorzugsweise ist die weitere Dichtung derart angeordnet, dass sie an einer korrespondierenden Dichtfläche der Trokarhülse anliegt, wenn die Dichtelementhalterung mit der Trokarhülse verbunden ist.

Vorteilhaft ist es, wenn die weitere Dichtung mit dem Halterungsdichtelement unlösbar verbunden ist. Auf diese Weise wird es insbesondere möglich, die Dichtelementhalterung mit nur einer Hand mit der Trokarhülse zu verbinden oder von dieser zu lösen. Vorzugsweise ist die weitere Dichtung an das Halterungsdichtelement angeformt, insbesondere angespritzt. Auf diese Weise kann die Dichtelementhalterung einfach und kostengünstig hergestellt werden. Eine besonders gute und sichere Abdichtung zwischen der Dichtelementhalterung und der Trokarhülse kann erreicht werden, wenn die weitere Dichtung aus einem Elastomer hergestellt ist.

Die eingangs gestellte Aufgabe wird bei einem chirurgischen Abdichtungssystem der eingangs beschriebenen Art erfindungsgemäß dadurch gelöst, dass das Halterungsdichtelement unter Vorspannung gegen eine Dichtfläche der Trokarhülse drückt eziehungsweise vorgespannt an der Dichtfläche anliegt. Durch das Halterungsdichtelement können auf einfache Weise Fluidverluste, insbesondere Gasverluste, durch das Abdichtungssystem hindurch verhindert werden. Ein durch die Trokarhülse definierter Instrumenteneinführkanal kann so optimal abgedichtet werden, zum einen durch das Halterungsdichtelement und zum anderen insbesondere durch ein in die Dichtelementhalterung eingesetztes Dichtelement. Die Dichtelementhalterung hat ferner den Vorteil, dass insbesondere Verschleißteile des Dichtungssystems, beispielsweise das Dichtelement, auf einfache Weise ausgetauscht werden können. Das Halterungsdichtelement kann zudem eine spielfreie Fixierung der Dichteinheit in der Trokarhülse oder einem Dichtungsgehäuse derselben sicherstellen. Des Weiteren lassen sich Fertigungstoleranzen der Dichtelementhalterung und/oder der Trokarhülse durch das Halterungsdichtelement einfach und sicher ausgleichen. Es werden keine weiteren Dichtungselemente benötigt, um die Dichtelementhalterung relativ zur Trokarhülse abzudichten. Dies vereinfacht sowohl die Montage als auch die Demontage der Dichtelementhalterung von der Trokarhülse. Des Weiteren können so auch Kosten des Abdichtungssystems insgesamt reduziert werden, da beispielsweise das Halterungsdichtelement direkt in die Dichtelementhalterung integriert werden kann.

Besonders vorteilhaft ist es, wenn die Dichtelementhalterung des Abdichtungssystems eine der oben beschriebenen Dichtelementhalterungen ist. Mit einer solchen Dichtelementhalterung verbessern sich die Handhabung und Bedienung auch des Abdichtungssystems insgesamt in der jeweils oben beschriebenen Weise.

Vorzugsweise ist die Dichtelementhalterung mit der Trokarhülse lösbar verbindbar. Dies ermöglicht es, die Dichtelementhalterung, an welcher insbesondere ein chirurgisches Dichtelement gehalten werden kann, bei einem am Dichtelement oder der Dichtelementhalterung aufgetretenen Verschleiß oder einer Beschädigung auf einfache Weise von der Trokarhülse zu lösen.

Vorzugsweise weist die Trokarhülse eine Dichtelementhalterungsaufnahme auf zum Einsetzen der Dichtelementhalterung. Es ist so möglich, die Teile des Abdichtungssystems einfach und sicher zusammenzusetzen und gegebenenfalls wieder auszutauschen.

Auf einfache und sichere Weise kann eine Abdichtung der Dichtelementhalterung relativ zur Trokarhülse erreicht werden, wenn das Halterungsdichtelement an einer in proximaler oder im Wesentlichen in proximaler Richtung weisenden Ringfläche der Trokarhülse anliegt. Insbesondere wird es so auf einfache Weise möglich, die Dichtelementhalterung mit der Trokarhülse mit nur einer Hand zu verbinden oder auch wieder von ihr zu lösen. Eine solche Einhandbedienung ist gerade bei in radialer Richtung wirkenden Dichtelementen, beispielsweise bei auf eine Außenfläche der Dichtelementhalterung aufgeschobenen Dichtringen, nicht möglich.

Günstig ist es, wenn die Ringfläche eine zusätzliche Dichtung trägt. Insbesondere dann, wenn das Halterungsdichtelement selbst nur wenig oder gar nicht elastisch oder flexibel ausgebildet ist, ermöglichst es die zusätzliche Dichtung, eine optimale Abdichtung relativ zur Dichtelementhalterung, insbesondere relativ zum Halterungsdichtelement, zu realisieren. Vorzugsweise ist die zusätzliche Dichtung derart angeordnet, dass sie am Halterungsdichtelement anliegt, wenn die Dichtelementhalterung mit der Trokarhülse verbunden ist.

Vorteilhaft ist es, wenn die zusätzliche Dichtung mit der Ringfläche unlösbar verbunden ist. Auf diese Weise wird es insbesondere möglich, die Dichtelementhalterung mit nur einer Hand mit der Trokarhülse zu verbinden oder von dieser zu lösen. Vorzugsweise ist die zusätzliche Dichtung an das Halterungsdichtelement angeformt, insbesondere angespritzt. Auf diese Weise kann die Trokarhülse einfach und kostengünstig hergestellt werden.

Eine besonders gute und sichere Abdichtung zwischen der Dichtelementhalterung und der Trokarhülse kann erreicht werden, wenn die zusätzliche Dichtung aus einem Elastomer hergestellt ist.

Besonders einfach wird der Aufbau des Abdichtungssystems, wenn die Ringfläche durch eine einstufige Durchmesserverjüngung eines Innendurchmessers der Trokarhülse definiert ist. Insbesondere kann die Durchmesserverjüngung in Richtung auf ein distales Ende der Trokarhülse hin ausgebildet sein. Sie kann jedoch auch schrittweise oder kontinuierlich verlaufen und gegenüber einer quer zur Längsachse der Trokarhülse verlaufenden Ebene geneigt sein, zum Beispiel um einen Neigungswinkel in einem Bereich von 10° bis 80°, vorzugsweise 30° bis 60°.

Um einen Schaft eines in den Trokar einführbaren Instruments gegen Gasverluste abzudichten, wenn der Instrumentenschaft für einen chirurgischen Eingriff die Trokarhülse durchsetzt, ist es günstig, wenn das Abdichtungssystem ein an der Dichtelementhalterung gehaltenes chirurgisches, eine erweiterbare Einführöffnung aufweisendes Dichtelement zum Abdichten der Einführöffnung beim Einführen eines chirurgischen Instruments umfasst.

Des Weiteren ist es günstig, wenn das Abdichtungssystem eine chirurgische Schutzvorrichtung für das Dichtelement umfasst, welche Schutzvorrichtung einen am Trokar oder an einem Teil desselben anordenbaren, ringförmig geschlossenen oder im Wesentlichen ringförmig geschlossenen und eine Durchbrechung aufweisenden Grundkörper mit mehreren in Umfangsrichtung angeordneten und parallel oder auf eine Längsachse der Schutzvorrichtung hin weisenden Schutzelemente umfasst, welche Schutzelemente im Wesentlichen in distaler Richtung weisende freie Enden aufweisen, wobei mindestens ein Teil der Schutzelementen an ihren freien Enden oder im Bereich ihrer freien Enden auf ihrer Außenseite mindestens ein Rückhalteelement zum in Eingriff Bringen mit dem Dichtelement aufweisen. Die Schutzelemente der Schutzvorrichtung können eine Innenfläche des Dichtelements im Wesentlichen vollständig verdecken, so dass beim Einführen eines Instruments in die Trokarhülse zunächst das Instrument mit den Schutzelementen in Kontakt tritt und diese erforderlichenfalls aufweitet, wobei dann die Schutzelemente mit dem Dichtelement in Kontakt treten und dieses aufweiten können. So können Verletzungen oder Beschädigungen des Dichtelements durch in die Trokarhülse eingeführte Instrumente vermieden werden. Die Rückhalteelemente an zumindest einigen der Schutzelemente vorzusehen, hat den Vorteil, dass es gezielt zu einem Verhaken der Schutzvorrichtung mit dem Dichtelement kommen kann, das beim Einführen eines Instruments gerade vermieden werden soll. Beim Einführen von Instrumenten hat das in Eingriff Bringen der Rückhalteelemente mit dem Dichtelement den Vorteil, dass so sichergestellt ist, dass ein Schutz des Dichtelements bis an den Bereich des Dichtelements heran, welcher am eingeführten Instrumentenschaft anliegt, also insbesondere eine definierte Dichtlinie oder Dichtlippe, sichergestellt werden kann. Durch das in Eingriff Bringen der Rückhalteelemente mit dem Dichtelement führt eine Aufweitung der Einführöffnung der Schutzvorrichtung automatisch auch zu einer Aufdehnung des Dichtelements. Eine Relativbewegung der Schutzvorrichtung und des Dichtelements wird jedoch im Wesentlichen verhindert. Dies hat den Vorzug, dass eine ab dem in Eingriff Bringen der Rückhalteelemente mit dem Dichtelement vorgegebene Bedeckung desselben in axialer Richtung der Schutzvorrichtung unabhängig von einer Einführstellung des angeführten Instruments sichergestellt werden kann. Ein in Eingriff Bringen kann insbesondere dann erfolgen, wenn das Rückhalteelement einen minimalen Durchmesser aufweist, so dass es dann, wenn es auf beispielsweise ein aus einem Elastomer hergestelltes Dichtelement trifft, dessen Wand ausbeulen und so quasi eine korrespondierende Ausnehmung definieren kann, in welche das Rückhalteelement eingreift. Durch das Ausbeulen wird jedoch ein Entlanggleiten des das Dichtelement ausbeulenden Gegenstands am Dichtelement verhindert. Vorzugsweise ist an den jeweiligen Schutzelementen ein einziges Rückhalteelement vorgesehen. Prinzipiell wäre es auch denkbar, mehrere Rückhalteelemente, also zwei, drei oder mehr an jeweils einem Schutzelement vorzusehen, um durch in Eingriff bringen der Rückhalteelemente mit dem Dichtelement eine Relativbewegung der beiden Teile beim Auffalten des Dichtelements zum Aufweiten der Einführöffnung zu verhindern.

Günstig ist es, wenn das mindestens eine Rückhalteelement in Form eines vom jeweiligen Schutzelement abstehenden Rückhaltevorsprungs ausgebildet ist. Derartige Rückhalteelemente sind besonders einfach herzustellen und können entsprechend dimensioniert werden, um ein gezieltes Verhaken oder anderweitiges in Eingriff Bringen der Rückhaltevorsprünge mit dem Dichtelement sicherzustellen.

Je nach Orientierung der Schutzelemente in einer Grundstellung oder auch einer aufgeweiteten Stellung ist es vorteilhaft, wenn mindestens ein Teil der Rückhaltevorsprünge senkrecht oder im Wesentlichen senkrecht von den Schutzelementen absteht. Zudem ist eine derartige Ausgestaltung der Rückhaltevorsprünge besonders einfach herzustellen. Ein Verhaken oder in Eingriff Bringen der Rückhaltevorsprünge mit dem Dichtelement kann insbesondere dann, wenn die Schutzelemente parallel zu einer vom Dichtelement definierten Längsachse am Grundkörper abstehen, verbessert werden, wenn mindestens ein Teil der Rückhaltevorsprünge schräg bezogen auf eine Erstreckung der Schutzelemente im Bereich ihrer freien Enden von diesen weg weisend absteht. Insbesondere weisen derartige Rückhaltevorsprünge vom Grundkörper weg und nach außen in Richtung auf das Dichtelement hin. So kann einfach und sicher ein Verhaken der Rückhaltevorsprünge mit dem Dichtelement sichergestellt werden.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Gesamtansicht eines chirurgischen Abdichtungssystems;
- Figur 2:: eine Schnittansicht längs Linie 2-2 in Figur 1;
- Figur 3:: eine vergrößerte Teilansicht der Schnittansicht in Figur 2;
- Figur 4:: eine perspektivische Explosionsdarstellung des Abdichtungssystems aus Figur 1;
- Figur 5:: eine perspektivische Ansicht des Dichtungselements aus Figur 4;
- Figur 6:: eine Schnittansicht längs Linie 6-6 in Figur 3;
- Figur 7:: eine perspektivische Explosionsdarstellung eines Dichtelements mit Schutzvorrichtung;
- Figur 8:: eine Schnittansicht längs Linie 8-8 in Figur 7;
- Figur 9:: eine Schnittansicht längs Linie 9-9 in Figur 7;
- Figur 10:: eine perspektivische Ansicht der Schutzvorrichtung in einer maximal aufgeweiteten Stellung;
- Figur 11:: eine Schnittansicht analog Figur 3 beim Einführen eines Obturators des Abdichtungssystems;
- Figur 12:: eine Ansicht analog Figur 1 des Abdichtungssystems mit eingesetztem Obturator;
- Figur 13:: eine Längsschnittansicht des in Figur 12 dargestellten Obturators; und

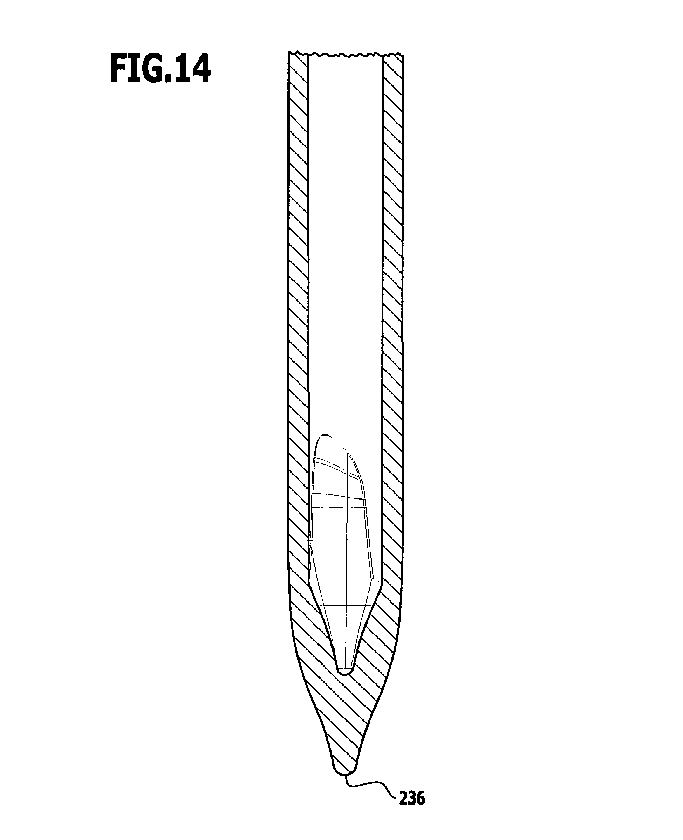

- Figur 14:: eine Schnittansicht längs Linie 14-14 in Figur 13.

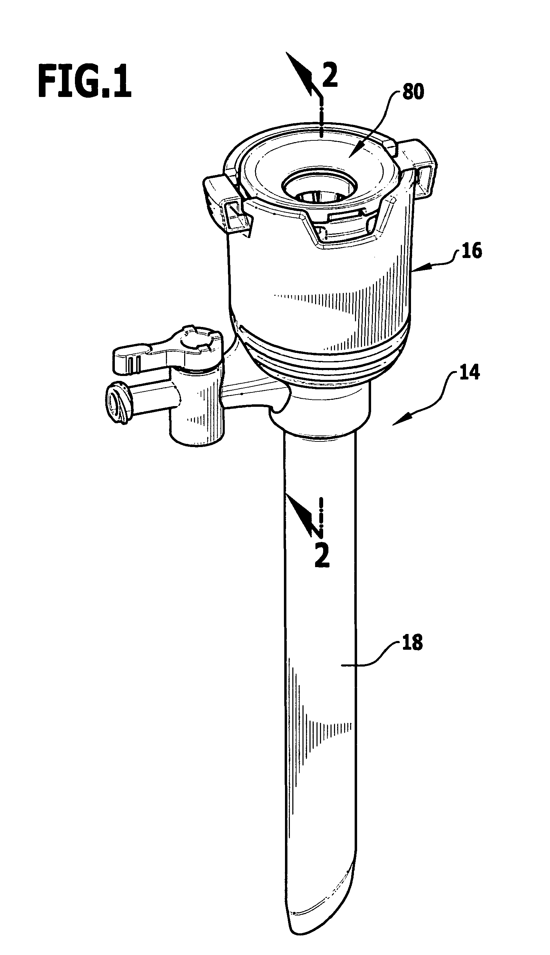

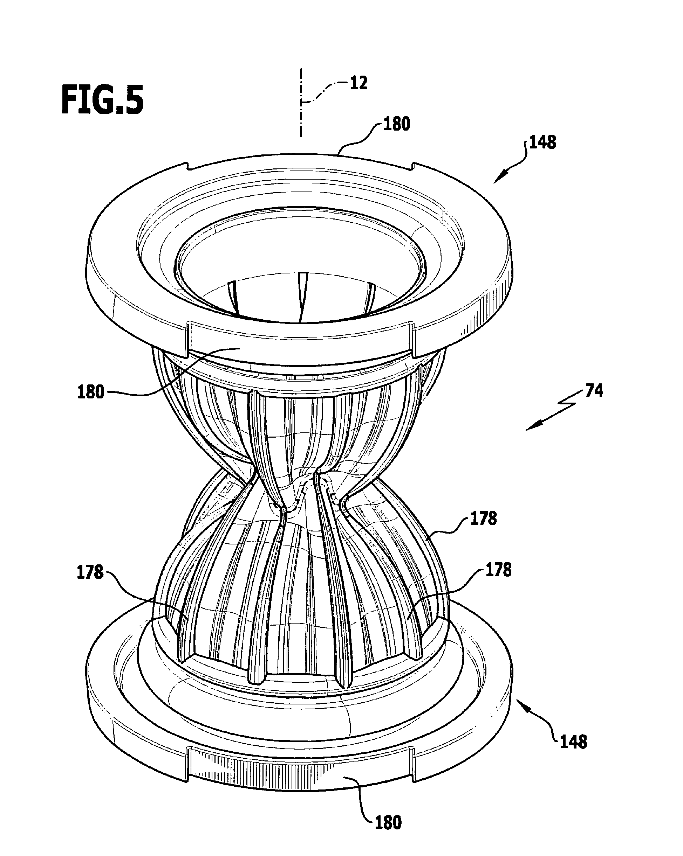

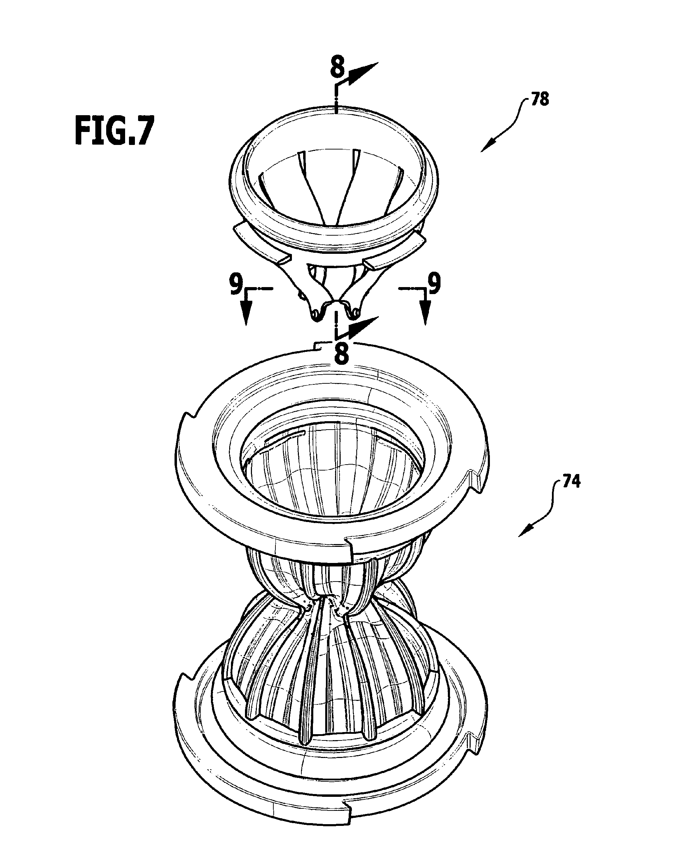

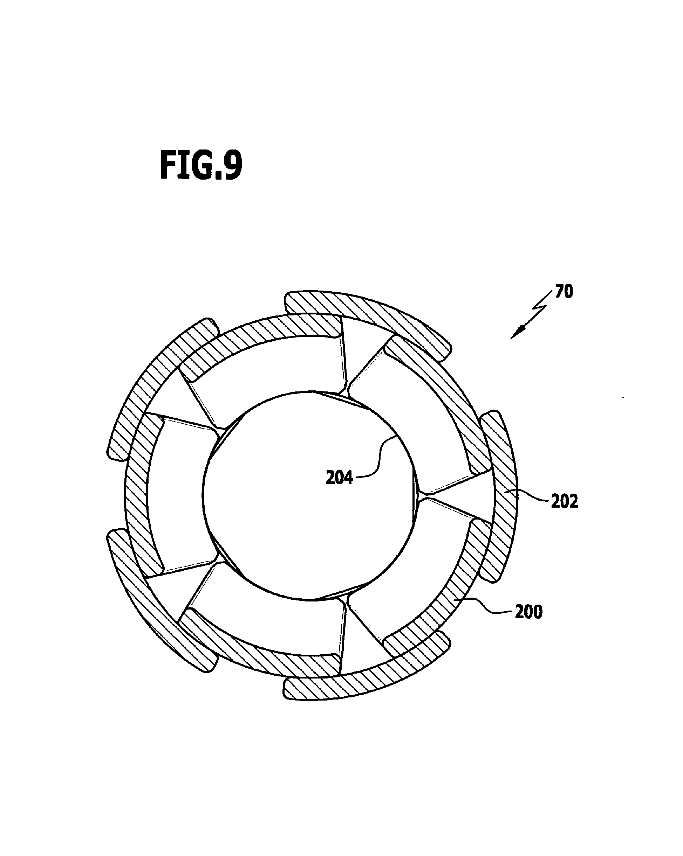

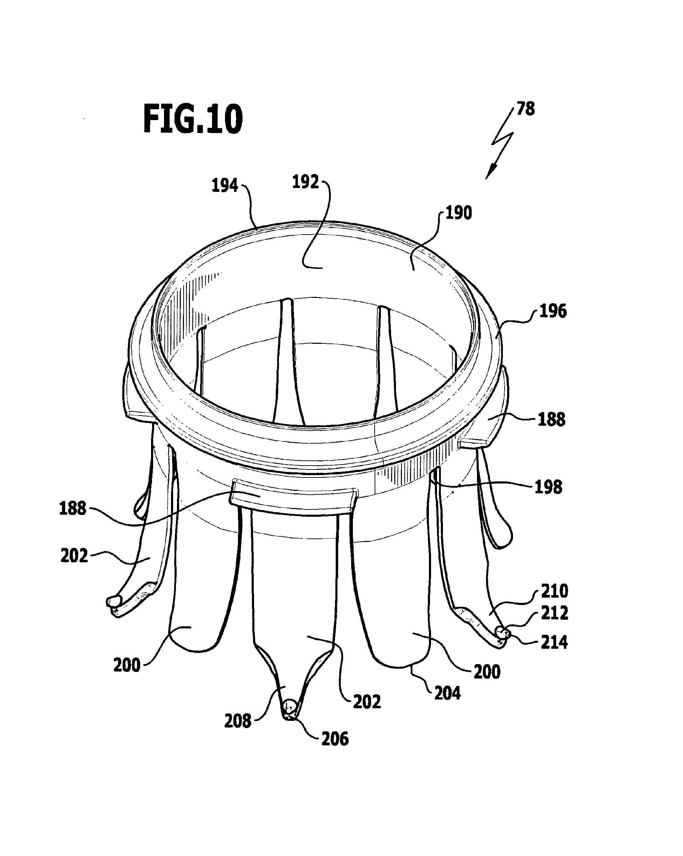

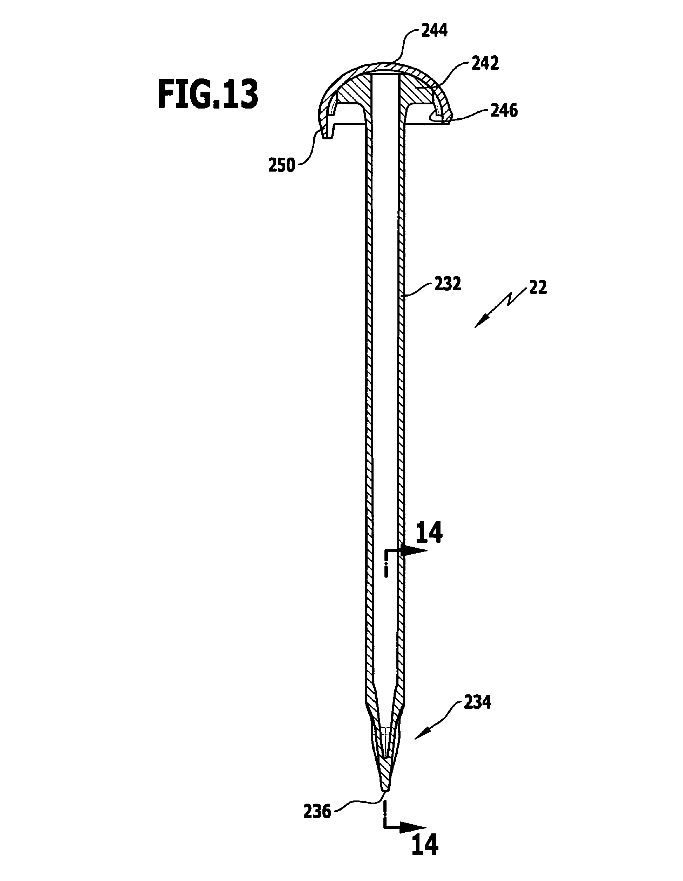

In den Figuren 1 bis 13 ist ein insgesamt mit dem Bezugszeichen 10 versehenes, ein chirurgisches Abdichtungssystem bildendes Trokarsystem dargestellt. Es umfasst eine eine Längsachse 12 definierende Trokarhülse 14 mit einem Dichtungsgehäuse 16 und einem sich von diesem weg in distaler Richtung erstreckenden Schaft 18, eine im Dichtungsgehäuse 16 angeordnete Dichtungsanordnung 20 sowie einen Obturator 22, welcher ein speziell zum Trennen und Aufweiten von Körpergewebe geformtes distales Ende aufweist und vor dem Einführen der Trokarhülse 14 in einen Patientenkörper in die Trokarhülse 14 eingeschobenen wird, um das Einführen der Trokarhülse 14 in den Patientenkörper zu Erleichtern.

Die Trokarhülse 14 ist im Wesentlichen rotationssymmetrisch ausgebildet und definiert im Inneren des Dichtungsgehäuses 16 eine Aufnahme 24 für die Dichtungsanordnung 20. Ein minimaler Innendurchmesser der Trokarhülse 14 wird definiert durch den Schaft 18. In einem ersten Übergangsbereich 26 vom Schaft 18 zum Dichtungsgehäuse 16 erweitert sich der Innendurchmesser des Schafts 18 kontinuierlich und bleibt im Bereich eines ersten Erweiterungsraums 28 konstant. An den ersten Erweiterungsraum 28 schließt sich ein zweiter Übergangsbereich 30 an, in dem sich der Innendurchmesser der Trokarhülse 14 nochmals kontinuierlich erweitert bis zu einem distalen Teil 32 der Aufnahme 24.

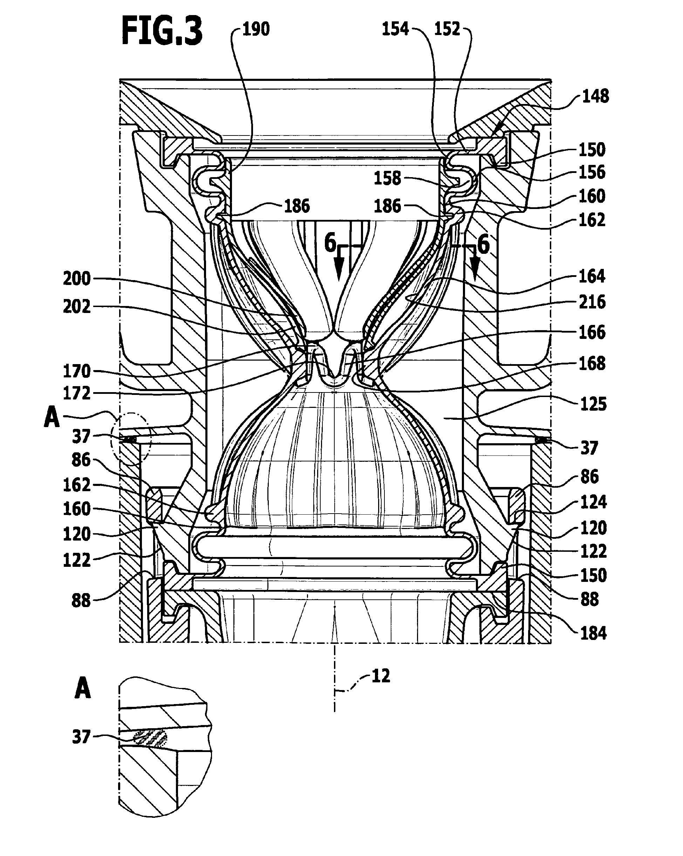

Ein Innendurchmesser des Dichtungsgehäuses 16 erweitert sich einstufig beim Übergang vom distalen Teil 32 zu einem proximalen Teil 34 desselben, so dass eine in proximaler Richtung weisende Ringfläche 36 definiert wird. Die Ringfläche kann optional eine zusätzliche Dichtung 37 tragen, welche durch Anspritzen eines Elastomers hergestellt und beispielhaft in Figur 3 punktiert eingezeichnet ist. Eine flache Ausnehmung 38 in der Ringfläche definiert somit eine etwas in proximaler Richtung vorstehende ebene Dichtfläche 40, welche von einer Innenwand 42 des proximalen Teils 34 durch die Ausnehmung 38 getrennt ist.

Ausgehend von einem proximalen Ende 44 des Dichtungsgehäuses 16 sind diametral bezogen auf die Längsachse 12 einander gegenüberliegend zwei zueinander symmetrische Verriegelungsaufnahmen 46 ausgebildet, welche jeweils zwei seitliche Hinterschneidungen 48 aufweisen, welche in Umfangsrichtung in einander entgegengesetzte Richtungen geöffnet sind. Die Verriegelungsaufnahmen 46 bilden einen Teil einer Rastverbindung, mit welcher die Dichtungsanordnung 20 im Dichtungsgehäuse 16 verriegelt werden kann, wie nachfolgend noch im Einzelnen noch erläutert wird.

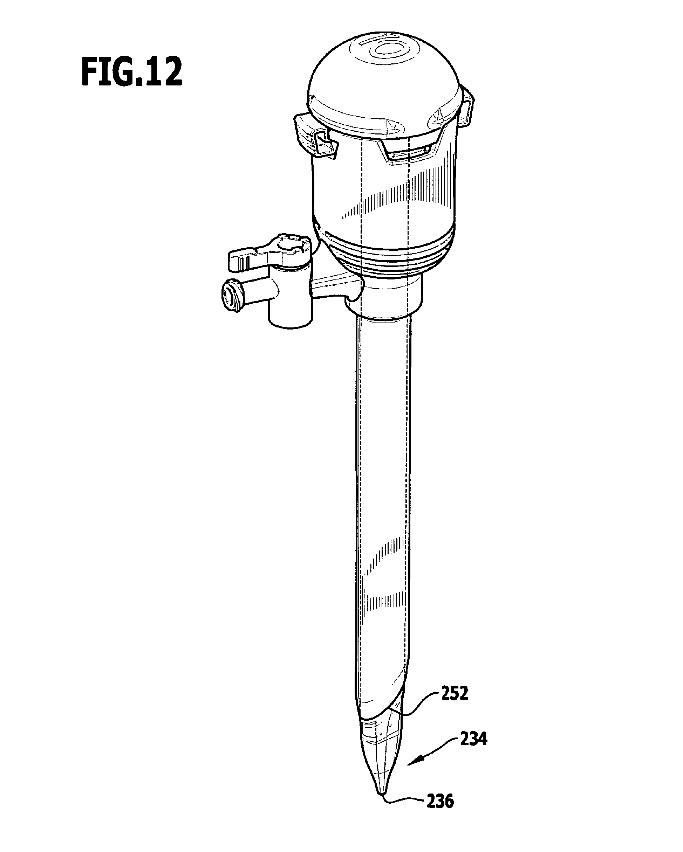

Ferner ist ausgehend vom Ende 44 in einer Wand 52 des Dichtungsgehäuses 16 eine sich in distaler Richtung etwas verjüngende Aussparung 50 symmetrisch zwischen den Verriegelungsaufnahmen 46 ausgebildet, in welche ein entsprechender Vorsprung 250 des Obturators 22 eingreift, wenn der Obturator 22 vollständig in die Trokarhülse 14 eingeführt ist, wie dies in Figur 12 dargestellt ist.

Am Dichtungsgehäuse 16 ist im Bereich des zweiten Übergangsbereichs 30 einseitig ein Anschlussstutzen 54 angeformt, welcher einen senkrecht zur Längsachse 12 verlaufenden Kanal 56 definiert. In den Kanal 56 eingesteckt ist ein Stutzen 57 eines Verschlusselements 58 mit einem in entgegengesetzter Richtung abstehenden, standardisierten Luer-Lock-Anschluss 60. Das Verschlusselement 58 umfasst ein zylindrisches Ventilgehäuse 62, in welchen ein korrespondierend ausgebildeter zylindrischer Verschlusskolben 64 mit einem angeformten Betätigungshebel 66 eingesetzt ist. Der Verschlusskolben 64 ist mit einer Bohrung 68 versehen, so dass in Abhängigkeit einer Verdrehstellung des Verschlusskolbens 64 relativ zum Ventilgehäuse 62 den Kanal 56 für Fluide geöffnet oder geschlossen werden kann. Statt des beschriebenen Verschlusselements 58 können auch beliebige andere Arten bekannter Verschlusselemente vorgesehen sein, insbesondere auch spezielle, federbelastete und dadurch selbstschließende Luer-Lock-Anschlüsse.

Die Dichtungsanordnung 20 umfasst zwei Dichtungen, nämlich ein Kreuzschlitzventil 70, welches an einem Haltering 72 gehalten ist, sowie ein Dichtelement 74, welches in seinem prinzipiellen Aufbau im deutschen Gebrauchsmuster 20 2006 005 442 detailliert beschrieben ist. Die dortige Beschreibung wird hiermit in vollem Umfang in die vorliegende Beschreibung mit einbezogen.

Das Dichtelement 74 ist im Inneren einer Dichtelementhalterung 76 gehalten, welche mit dem Haltering 72 lösbar verbindbar ist. Proximalseitig ist am Dichtelement 74 eine Schutzvorrichtung 78 lösbar gehalten, so dass das Dichtelement 74 zusammen mit der Schutzvorrichtung 78 bei Bedarf aus der Dichtelementhalterung 76 entnommen werden kann. Proximalseitig kann die Dichtelementhalterung 76 noch mit einem Deckel 80 verschlossen werden.

Die einzelnen Teile der Dichtungsanordnung 20 werden nachfolgend im Einzelnen näher beschrieben.

Der Haltering 72 umfasst einen im Querschnitt kreisförmigen Ring 82, von dessen proximalseitigem Rand ein in proximaler Richtung abstehender ringförmiger Flansch 84 ausgebildet ist, welcher sich jedoch nicht über eine gesamte Breite einer Wand des Rings 82 erstreckt, sondern nur etwa über die Hälfte.

Vom proximalen Rand des Rings 82 stehen ferner zwei zueinander symmetrisch ausgebildete Verbindungsflügel 86 einander, bezogen auf die Längsachse 12, diametral gegenüberliegend in proximaler Richtung ab. Die Verbindungsflügel 86 weisen jeweils zwei im Wesentlichen rechteckige Durchbrechungen 88 auf, welche quer zur Längsachse 12 orientiert sind. Die Verbindungsflügel 86 sind vom Flansch 84 etwas beabstandet angeordnet, so dass zwischen dem Flansch 84 und den Verbindungsflügeln 86 jeweils eine Nut 90 ausgebildet ist.

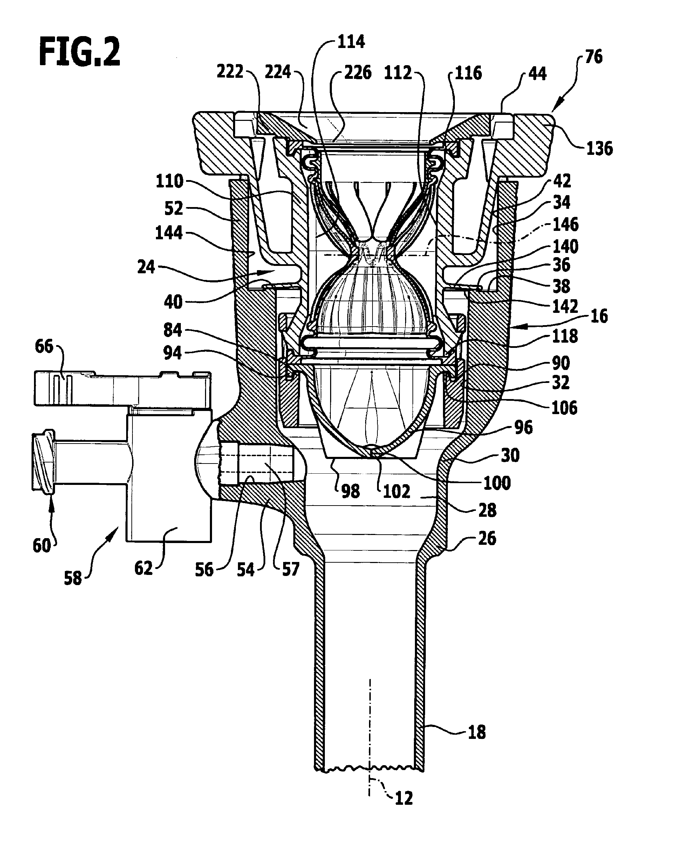

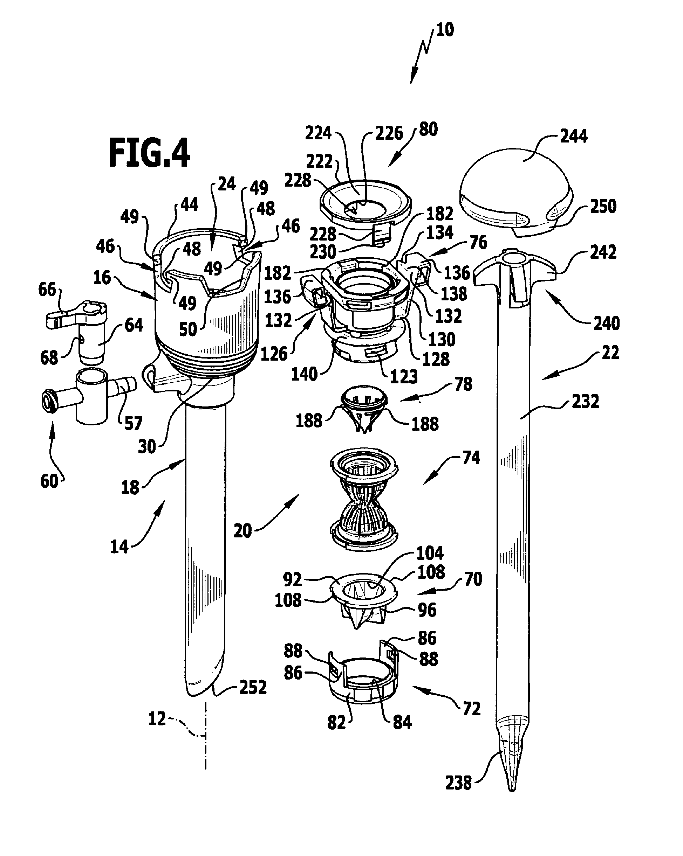

Das Kreuzschlitzventil 70 umfasst proximalseitig einen ringförmigen Befestigungsflansch 92, welcher einen in distaler Richtung weisenden Ringvorsprung 94 trägt, welcher in seiner Höhe sowie in seinen äußeren Abmessungen korrespondierend zu den Nuten 90 ausgebildet ist. Das Kreuzschlitzventil 70 umfasst ferner einen am Befestigungsflansch 92 in distaler Richtung abstehenden Ventilkörper 96, welcher distalseitig in eine kreuzförmige Endfläche 98 mündet, die mit zwei zueinander senkrechten Schlitzen 100 versehen ist. Der Ventilkörper 96 ist in einer Grundstellung, wie sie beispielsweise in den Figuren 2 und 4 dargestellt ist, so ausgebildet, dass die durch die Schlitze 100 getrennten Schnittflächen 102 des Ventilkörpers 96 direkt aneinander anliegen und so eine durch den Befestigungsflansch 92 definierte ringförmige Öffnung 104 etwas distalseitig des Befestigungsflanschs 92 vollständig verschließen. Der Ventilkörper 96 ist proximalseitig direkt an einen Innenrand des Befestigungsflansch 92 angeformt, so dass zwischen dem Ventilkörper 96 und dem Ringvorsprung 94 eine Ringnut 106 ausgebildet wird, in welche der Flansch 84 im Wesentlichen formschlüssig eingreifen kann.

Der Befestigungsflansch 92 ist ferner mit zwei in radialer Richtung weisenden Aussparungen 108 versehen, in welche die Verbindungsflügel 86 eingreifen, wenn der Ventilkörper 96 in den Haltering 72 eingesetzt ist und mindestens teilweise mit dem Ventilkörper 96, insbesondere mit dessen die Schlitze 100 aufweisender Endfläche 98, über einen distalseitigen Rand des Rings 82 vorsteht.

Die Dichtelementhalterung 76 ist im Wesentlichen langgestreckt hülsenförmig ausgebildet. Sie umfasst einen zentralen, koaxial zur Längsachse 22 ausgebildeten Hülsenkörper 110. Eine Innenfläche 112 des Hülsenkörpers 110 ist vollständig rotationssymmetrisch ausgebildet. Die Innenfläche 112 definiert und begrenzt einen Längskanal 114, in welchen das Dichtelement 74 eingesetzt ist. Ein Innendurchmesser des Hülsenkörpers 110 erweitert sich jeweils zum distalen und proximalen Ende desselben hin etwas. Distalseitig und proximalseitig sind jeweils ringförmige, in proximaler beziehungsweise distaler Richtung weisende Ringvorsprünge 116 und 118 ausgebildet, zum in Eingriff Bringen mit korrespondierenden Flanschen beziehungsweise Nuten am Dichtelement 74.

Auf einer Außenseite des Hülsenkörpers 110 sind etwas proximalseitig des distalseitigen Ringvorsprungs 118 zwei bezogen auf die Längsachse 12 einander diametral gegenüberliegende und in entgegengesetzte Richtungen weisende Rastnasen 120 ausgebildet, welche nach außen weisende, etwas in distaler Richtung geneigte Aufgleitflächen 122 und somit auch eine in proximaler Richtung weisende Ringkante 124 definieren. Die Rastnasen 120 sind korrespondierend zu den Durchbrechungen 88 an den Verbindungsflügeln 86 ausgebildet. Die Verbindungsflügel 86 können von distal her kommend über die Aufgleitflächen 122 geschoben werden, so dass sie etwas in radialer Richtung von der Längsachse 12 weg nach außen ausschwenken. Sobald die Rastnasen 120 ganz in die Durchbrechungen 88 eingreifen können, federn die Verbindungsflügel 86 wieder in Richtung auf die Längsachse 12 zurück. Auf die beschriebene Weise können der Haltering 72 und die Dichtelementhalterung 76 rastend miteinander verbunden werden.

Zwischen den Rastnasen 120, also um 90° relativ zu diesen in Umfangsrichtung versetzt, sind im Hülsenkörper 110 zwei rechteckige Durchbrechungen 123 vorgesehen, welche einen Innenraum 125 des Hülsenkörpers 110 mit einer Außenseite desselben verbinden. Auf diese Weise kann ein Druckausgleich zwischen dem Innenraum 125 und einer Umgebung der Dichtelementhalterung 76 erreicht werden. Der so erreichbare Druckausgleich zwischen einem im Körper eines Patienten herrschenden Gasdrucks und dem Innenraum 125 bzw. die so mögliche Ent-/Belüftung des Innenraums 125 verhindert, dass eine Aufweitung des Dichtelements 74 gegen ein Gasvolumen im Innenraum erfolgen muss, welches nach Montage des Dichtelements 74 an der Dichtelementhalterung 76 eingeschlossen würde.

Zum Verbinden der Dichtelementhalterung 76 mit dem Dichtungsgehäuse 16 sind von einer Außenseite der Dichtelementhalterung 76 zwei Kupplungsglieder 126 einander diametral gegenüberliegend abstehend angeordnet. Sie umfassen jeweils einen direkt vom Hülsenkörper 110 in radialer Richtung abstehenden Quersteg 128, von welchem sich ein im Wesentlichen parallel zum Hülsenkörper 110 in proximaler Richtung erstreckender Federteil 130 weg erstreckt. An einem proximalen Ende des Federteils 130 sind beidseits des Federteils 130 im Wesentlichen in Umfangsrichtung weisende und überstehende Rastvorsprünge 132 ausgebildet, welche jeweils von der Längsachse 12 weg weisende Aufgleitflächen 134 definieren. Zwischen den Aufgleitflächen 134 ist auf einer Außenseite der Federteile 130 ein im Wesentlichen quaderförmiges Bedienelement 136 angeordnet, welches proximalseitig etwas über das Ende des Federteils 130 vorsteht.

Zum Verbinden der Dichtelementhalterung 76 mit dem Dichtungsgehäuse 16 wird das distale Ende der Dichtelementhalterung 76 in das Dichtungsgehäuse 16 eingeführt, bis die die Hinterschneidungen 46 seitlich begrenzende Vorsprünge 49 mit den Aufgleitflächen 134 in Kontakt kommen und die Federteile 130 infolge des Aufgleitens etwas in Richtung auf die Längsachse 12 hin verschwenken. Sobald eine proximale Endfläche 138 der Federteile 130 in die Hinterschneidung 48 eintauchen kann, federn die Federteile 130 in radialer Richtung etwas nach außen und die Endfläche 130 liegt an einer in distaler Richtung weisenden Kante des Vorsprungs 49 an. Zum Lösen der Dichtelementhalterung 76 von der Trokarhülse 14 können die Bedienelemente 136 mit einer in Richtung auf die Längsachse 12 wirkenden Kraft beaufschlagt werden, so dass die Federteile 130 in Richtung auf die Längsachse 12 verschwenkt werden und die Rastvorsprünge 132 wieder die Hinterschneidung 48 freigeben. Die Dichtelementhalterung 76 kann dann in proximaler Richtung aus dem Dichtungsgehäuse 16 herausgezogen werden.

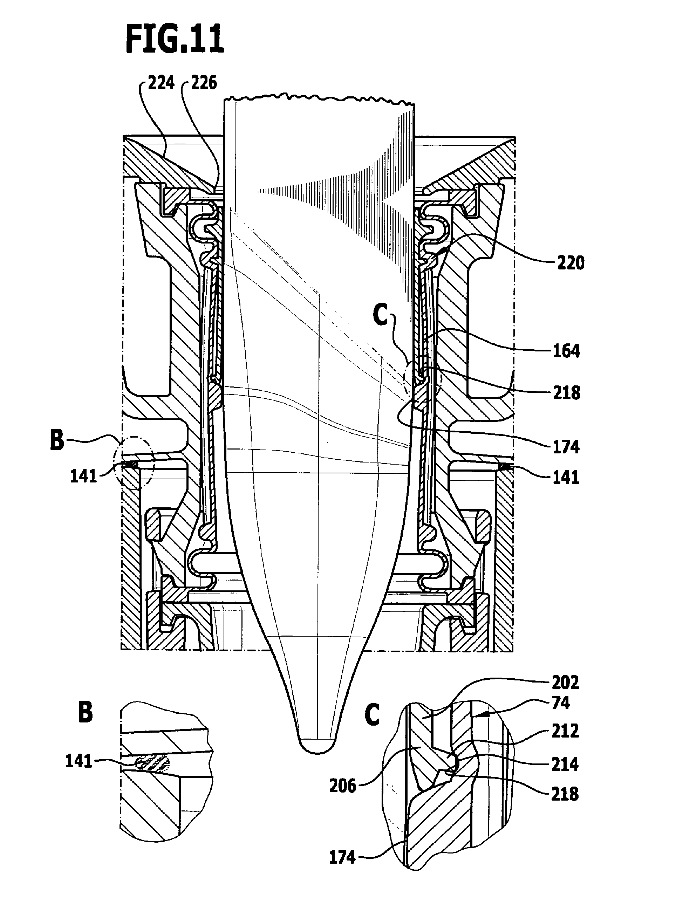

Etwas distalseitig der Querstege 128 ist ein Halterungsdichtelement 140 ausgebildet, und zwar in Form eines im Wesentlichen in radialer Richtung abstehenden Ringflansches, welcher etwas in distaler Richtung geneigt ist, und zwar um etwa 2° bezogen auf eine senkrecht zur Längsachse 12 verlaufende Querebene. Das Halterungsdichtelement 140 weist eine Dicke auf, welche eine gewisse Elastizität beziehungsweise Flexibilität des Halterungsdichtelements 140 vorgibt. Es kann so in axialer Richtung etwas federn und Fertigungstoleranzen an der Trokarhülse 14 und der Dichtelementhalterung 76 ausgleichen. Das Halterungsdichtelement 140 ist an der Dichtelementhalterung 76 derart angeordnet, dass dann, wenn die Dichtelementhalterung 76 rastend in der beschriebenen Weise mit dem Dichtungsgehäuse 16 verbunden ist, eine in distaler Richtung weisende Dichtfläche 142 der Dichtelementhalterung 76 an der Ringfläche 36, etwas vorgespannt, anliegt und so eine perfekte Abdichtung der Dichtelementhalterung 76 bezogen auf eine Innenwand 144 des Dichtungsgehäuses 16 der Trokarhülse erreicht wird. Das Halterungsdichtelement 140 kann zur Verbesserung einer Abdichtungswirkung optional eine weitere Dichtung 141 tragen, welche durch Anspritzen eines Elastomers hergestellt und beispielhaft in Figur 11 punktiert eingezeichnet ist.

Das Dichtelement 74 ist bezogen auf die Längsachse 12 im Wesentlichen rotationssymmetrisch ausgebildet. Ferner ist es bezogen auf eine quer zur Längsachse verlaufende Öffnungsebene 146 im Wesentlichen spiegelsymmetrisch ausgebildet. Die Öffnungsebene 146 verläuft parallel zu zwei beidseitigen Flanschringen 148, welche das Dichtelement 74 distal- und proximalseitig begrenzen und einen maximalen Außendurchmesser des Dichtelements 74 definieren. Von den Flanschringen 148 stehen maximal weit außen an ihnen und jeweils in die Richtung des anderen Flanschrings 148 hin weisend Ringvorsprünge 150 ab, welche die Ringvorsprünge 116 und 118 außen umgreifen können. Das Dichtelement 74 kann so auf einfache Weise über die Ringvorsprünge 116 und 118 gehängt oder gespannt und im Inneren der Dichtelementhalterung 76 gehalten werden.

Von den Flanschringen 148 erstreckt sich in radialer Richtung auf die Längsachse 12 hin ein erster Querabschnitt 152, welche in einen ersten, nach außen zurückgebogenen ersten Wulstabschnitt 154 übergeht, welcher wiederum direkt in einen zweiten Wulstabschnitt 156 übergeht, welcher wiederum ein auf die Längsachse 12 hin gerichtetes Ende aufweist. Der zweite Wulstabschnitt 156 definiert so eine in Richtung auf die Längsachse hin geöffnete Ringnut 158.

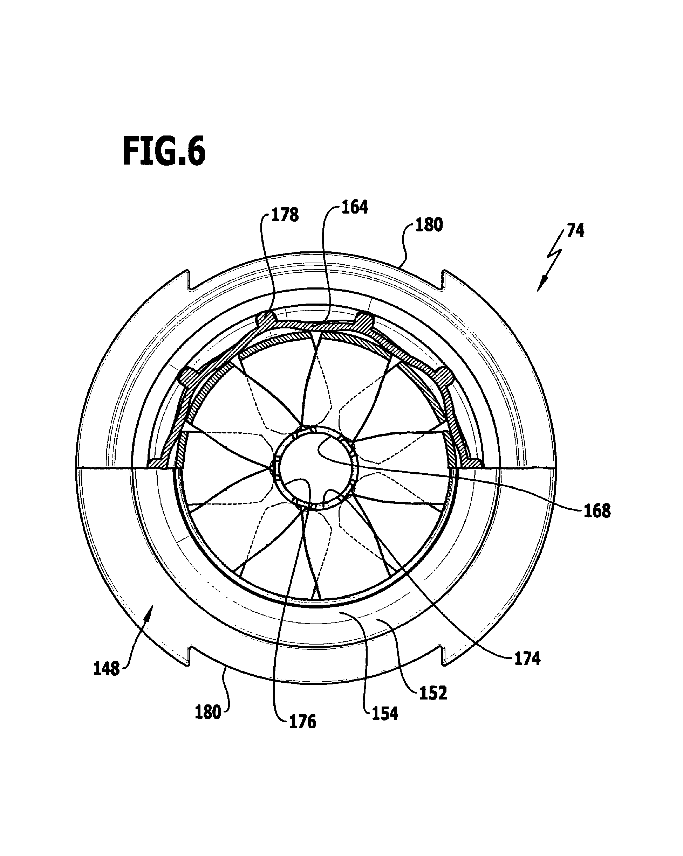

Distalseitig schließt sich an den zweiten Wulstabschnitt 156 ein kurzer zylindrischer Abschnitt 160 an, welcher in einen verdickten, außen am Dichtelement 74 abstehenden Wulst 162 übergeht. Ausgehend von den Wulsten 162, an denen eine Wand 164 des Dichtelements 74 im Wesentlichen faltenfrei absteht, faltet sich die Wand 164 gardinenartig bis zur Öffnungsebene 146 hin. Durch die Faltung entsteht eine Dichtlinie 166, welche in Form einer Wellenlinie Wellenberge 170 proximalseitig der Öffnungsebene 146 und Wellentäler 172 distalseitig der Öffnungsebene 146 definiert. Die Wellenlinie 168 ist etwas verstärkt und in Form einer Dichtlippe 174 ausgebildet, welche sich somit teilweise proximalseitig und teilweise distalseitig der Öffnungsebene 146 befindet. In einer Draufsicht, wie in Figur 6 dargestellt, ist jedoch erkennbar, dass die Dichtlinie 166 und damit auch die Dichtlippe 174 eine kreisringförmige Öffnung 176 des Dichtelements 74 begrenzen. Die Öffnung 176 weist in einer Grundstellung, wie sie beispielsweise in den Figuren 3 bis 6 dargestellt ist, einen minimalen Innendurchmesser auf. Die Öffnung 176 kann, wie beispielsweise in Figur 11 dargestellt, so weit aufgeweitet werden, dass ein Innendurchmesser derselben einem Innendurchmesser des Dichtelements 74 im Bereich der Wulste 162 entspricht. Die gefaltete Wand 164 entfaltet sich dabei, ist praktisch auf der gesamten Länge zwischen den Wulsten 162 vollständig umfaltet und definiert so eine im Wesentlichen zylindrische Wandfläche.

Zur Stabilisierung des Dichtelements 74 sind auf einer Außenseite der Wand 164 ausgehend von den Wulsten 164 bis an die Dichtlippe 166 heranreichende Verstärkungsrippen 178 ausgebildet. Das Dichtelement 74 ist insgesamt einstückig aus einem Kunststoff gespritzt, welcher vorzugsweise elastomerische Eigenschaften aufweist. Ferner sind an den Flanschringen 148 jeweils zwei, einander diametral gegenüberliegende Aussparungen 180 vorgesehen, welche korrespondierend zu zwei in radialer Richtung von der Dichtelementhalterung 76 in Richtung auf die Längsachse 12 hin vorstehenden Vorsprüngen 182 ausgebildet sind. Die Aussparungen 180 in Verbindung mit den Vorsprüngen 182 bilden eine Verdrehsicherung, so dass das Dichtelement 74 und die Dichtelementhalterung 76 in einer beispielsweise in den Figuren 2 und 3 dargestellten Montagestellung nicht um die Längsachse 12 relativ zueinander verdrehbar sind.

Nach Bestücken des Halterings 72 mit dem Kreuzschlitzventil 70 kann die Dichtelementhalterung 76, in welche das Dichtelement 74 in der oben beschriebenen Weise eingesetzt ist, mit dem Haltering 72 verbunden werden. Distalseitig bildet eine in distaler Richtung weisende ringförmige Stirnfläche 184 des Dichtelements 74 ein Anlagefläche für den Befestigungsflansch 92. Durch rastendes Verbinden des Halterings 72 mit der Dichtelementhalterung 76 in der oben beschriebenen Weise, werden der Befestigungsflansch 92 und der Flanschring 148 gegeneinander gedrückt und bilden eine perfekte Abdichtung.

Zumindest im proximalseitigen Wulst 162 sind vier gleichmäßig über den Umfang verteilte, in radialer Richtung auf die Längsachse 12 hin geöffnete und Verbindungsglieder bildende Vertiefungen 186 vorgesehen, welche der Aufnahme korrespondierender Verbindungselemente 188 der Schutzvorrichtung 78 dienen. Die Schutzvorrichtung 78 umfasst einen ringförmigen, in sich geschlossenen Grundkörper 190, welcher eine kreisförmige Durchbrechung 192 definiert. Vom Grundkörper 190 steht ein in radialer Richtung nach außen weisend benachbart einem proximalen Ende 194 ein Ringvorsprung 196 ab. Etwas weiter distalseitig sind die Verbindungselemente 188 in Form kurzer stegartiger Vorsprünge angeordnet. Sie erstrecken sich im Umfang über etwa 1/8 des Gesamtumfangs des Grundkörpers 190 und sind korrespondierend zu den Vertiefungen 186 ausgebildet. Der Grundkörper 190 kann somit direkt am Dichtelement 74 gelagert werden, wobei hierfür die Verbindungselemente 188 formschlüssig in die Vertiefungen 168 eingreifen. Sie bilden somit gleichzeitig eine Verdrehsicherung der Schutzvorrichtung 78 relativ zum Dichtelement 74. Ferner bilden sie auch eine Positionierhilfe der Schutzvorrichtung 78 relativ zum Dichtelement 74.

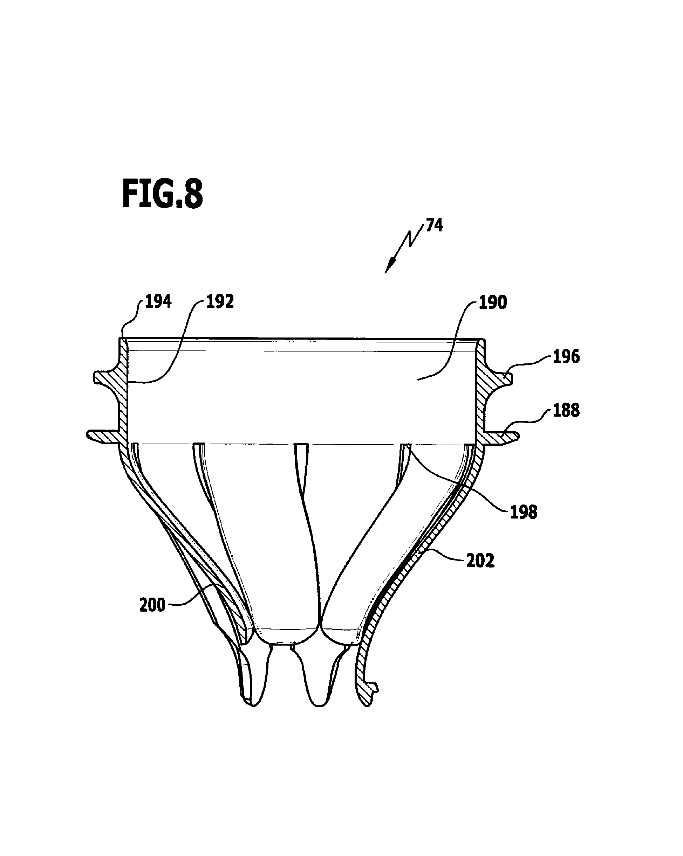

Von einem distalseitigen Rand 198 des Grundkörpers 190 erstrecken sich insgesamt 10, jeweils fünf lamellenförmige kurze Schutzelemente 200 und fünf lange Schutzelemente 202 in distaler Richtung. Sie weisen im Längsschnitt, wie in Figur 8 dargestellt, eine über ihre gesamte Länge konstante Dicke auf. Die kurzen Schutzelemente 200 sind im Wesentlichen bis zu ihrem freien Ende 204 nahezu gleich breit, die langen Schutzelemente 202 in etwa auf derselben Länge wie die kurzen Schutzelemente 200, allerdings nimmt dann eine Breite der langen Schutzelemente 202 auf deren distales Ende 206 hin deutlich ab, so dass ein schmaler Schutzelementabschnitt 208 ausgebildet wird, welcher in seiner Außenkontur im Wesentlichen korrespondierend zu einem Wellental 172 ausgebildet ist.

Die langen Schutzelemente 202 tragen jeweils ein von einer Außenseite 210 etwas in distaler Richtung geneigt abstehendes Rückhalteelement 212, welches eine Länge von weniger als 1mm aufweist. Das Rückhalteelement ist im Wesentlichen kegelstumpfförmig ausgebildet und weist eine abgerundete Spitze 214 auf.

Wenn der Grundkörper 190 in der oben beschriebenen Weise mit dem Dichtelement 174 verbunden ist, falten sich die aufgrund ihrer geringen Dicke flexiblen Schutzelemente 200 und 202 in Richtung auf die Längsachse 12 hin und nehmen die in den Figuren 6 bis 8 dargestellte Stellung ein. Es sei angemerkt, dass sich die langen Schutzelemente 202 distalseitig der Verbindungselemente 188 vom Rand 198 weg erstrecken, die kurzen Schutzelemente 200 in den Bereichen des Rands 198, zu welchem kein Verbindungselement 188 korrespondiert. Durch die entsprechend vorgesehenen Vertiefungen 186 kann die Schutzvorrichtung 78 positionsrichtig mit dem Dichtelement 74 verbunden werden, dies bedeutet, dass in einer Grundstellung alle fünf Schutzelementabschnitte 208 in korrespondierende Wellentäler 172 eintauchen. Damit ist sichergestellt, dass die distalen Enden 204 und 206 der Schutzvorrichtung 78 praktisch bis an die Dichtlinie 166 heranreichen und im Wesentlichen eine innere Wandfläche 216 des Dichtelements 74 vollständig verdecken.

Die Schutzelemente 200 und 202 stehen in der montierten Grundstellung bereits wenig distalseitig des Wulsts 162 von der Wandfläche 216 ab und berühren diese allenfalls nahe ihrer Enden 204 und 206. In der Grundstellung sind die Schutzelemente 200 und 202 einander überlappend angeordnet, wobei die kurzen Schutzelemente 200 näher an der Längsachse 12 liegen als die langen Schutzelemente 202. Dadurch berühren lediglich die Rückhalteelemente 212 benachbart der Dichtlinie 166 die Wand 164 des Dichtelements 74.

Wird ein Instrument, oder wie beispielsweise in Figur 11 dargestellt, der Obturator 22, von proximal her kommend in das Dichtelement 74 eingeführt, so tritt es zunächst in Kontakt mit Innenflächen der kurzen Schutzelemente 200. Ist ein Außendurchmesser, wie dies beim Obturator 22 der Fall ist, eines Instruments größer als die Öffnung 176, dann werden die kurzen Schutzelemente 200 gegen die langen Schutzelemente 202 gedrückt und nach außen verschwenkt. Dabei werden auch die Rückhalteelemente 212 mit ihren Spitzen 214 in die Wand 164 des Dichtelements 74 eingedrückt. Dies führt zu einer Auswölbung 218 der Wand 164 durch die Rückhalteelemente 212, so dass sich diese in der Wand 164 verhaken, man kann auch sagen, die Rückhalteelemente 212 und das Dichtelement 74 stehen miteinander in Eingriff. Durch das Verhaken der Rückhalteelemente 212 in der Wand 164 wird eine Relativbewegung der distalen Enden 206 der langen Schutzelemente 202 relativ zum Dichtelement 74 praktisch verhindert. Unabhängig von einer Aufweitung oder Auffaltung der Wand 164 in Abhängigkeit eines Durchmessers des eingeführten Instruments reichen die distalen Enden 206 der langen Schutzelemente 202 stets bis an die Dichtlinie 166 heran und schützen das Dichtelement 74 vor den eingangs beschriebenen möglichen Beschädigungen infolge des in Kontakt Tretens der Wand 164 mit spitzen Kanten der eingeführten Instrumente.

Selbst dann, wenn eine Längsachse des eingeführten Instruments relativ zur Längsachse 12 etwas verkippt wird, bleibt der Verhakeeffekt der Rückhalteelemente 212 erhalten. Durch eine von den Vertiefungen 186 und den Verbindungselementen 188 ausgebildete Verbindungseinrichtung 220 wird eine Verkippung des zunächst an der Schutzvorrichtung anliegenden Instrumentenschafts direkt auf das Dichtelement 74 übertragen, und zwar im Bereich des Wulstes 162, so dass das Dichtelement 74 analog zu einer Verkippung der Schutzvorrichtung 78 mit verkippt wird. Die besondere Anordnung der Schutzvorrichtung 78 am Dichtelement 74 bildet somit quasi auch eine Neigungsanpassung bei der Einführung von Instrumenten. Hierfür dienlich ist zudem insbesondere der erste Wulstabschnitt 154, welcher sowohl eine Verkippbewegung als auch eine transversale Bewegung, zumindest soweit der erste Wulstabschnitt 154 von einer Innenwand der Dichtelementhalterung 76 beabstandet ist, gestattet.

Durch die Wölbung der Schutzelemente 200 und 202 schwach konvex von der Wand 164 weg, ist sichergestellt, dass ein eingeführtes Instrument zunächst mit distalen Endbereichen der Schutzelemente 200 und 202 in Kontakt tritt, bevor es die Dichtlippe 174 berühren kann.

Zum Verschließen des Dichtungsgehäuses 16 dient der Deckel 80. Er umfasst einen ringförmigen Rahmen 222, von dem sich im Innern und in distaler Richtung eine sich im Durchmesser konisch verjüngende Deckelfläche 224 bis zu einer Deckelöffnung 226 erstreckt, welche einen maximalen Innendurchmesser der Dichtungsanordnung 20 definiert. Instrumente mit Schaftdurchmessern, welche größer sind als ein Innendurchmesser der Deckeöffnung 226, können nicht in die Trokarhülse 14 eingeführt werden. Der Deckel 80 weist ferner zwei in distaler Richtung weisende, einander gegenüberliegende Laschen 228 auf, welche an freien Enden Rastvorsprünge 230 aufweisen, welche mit korrespondierenden, in den Figuren nicht dargestellten Rastkanten an der Dichtelementhalterung 76 in Eingriff gebracht werden können. Der Deckel 80 kann dann nach Montage der Dichtelementhalterung 76 am Dichtungsgehäuse 16 auf einfache Weise auf die Dichtelementhalterung 76 aufgeschnappt werden.

Um die Trokarhülse 14 in einen menschlichen oder tierischen Körper einführen zu können, ist der Obturator 22 vorgesehen. Er umfasst einen hohlen, koaxial zur Längsachse 12 verlaufenden Schaft 232, welcher sich in einem distalen Endbereich 234 im Außendurchmesser kontinuierlich verjüngt und eine abgerundete Spitze 236 definiert. Der Endbereich 234 ist im Querschnitt an keiner Stelle kreisförmig, sondern in Folge definierter, sich parallel zur Längsachse 12 erstreckender Vertiefungen 238 unsymmetrisch ausgebildet. In einem proximalen Endbereich 240 sind an einer Außenseite des Schafts 232 vier jeweils 90° zueinander versetzt angeordnete Haltevorsprünge 242 vorgesehen, welche der Lagerung und Verbindung eines im Wesentlichen die Form einer Halbkugel aufweisenden Deckels 244 dienen. Am Deckel 244 sind auf einer Innenseite korrespondiere Vorsprünge 246 ausgebildet. Optional kann der Deckel 244 auch mit dem Schaft 232 und dessen Haltevorsprüngen 242 verschraubt oder verklebt sein. Des Weiteren steht vom Deckel 244 in distaler Richtung weisend der lappenförmige Vorsprung 250 ab, welcher korrespondierend zur Aussparung 50 ausgebildet ist, so dass der Obturator 22 mit einer definierten Orientierung bezogen auf die Längsachse 12 in die Trokarhülse 14 eingesetzt werden kann. Ist der Obturator 22 vollständig in die Trokarhülse 14 eingeschoben, steht der distale Endbereich 234 über eine bezogen auf die Längsachse 12 um etwa 45° geneigte Endfläche 252 des Schafts 18 vor, wie dies in Figur 12 dargestellt ist.

Im Inneren des Dichtungsgehäuses 16 erfolgt eine Abdichtung über das Dichtelement 74 sowie zu einem Außenbereich der Dichtelementhalterung 76 und der Innenwand 144 der Trokarhülse 14 mittels des Halterungsdichtelements 140. Wird der Obturator 22 aus der Trokarhülse 14 entfernt, verschließt das Kreuzschlitzventil 70 einen sich längs der Trokarhülse 14 erstreckenden Kanal fluiddicht. Aufgrund der etwas von der Längsachse 12 weg weisenden Außenflächen des Ventilkörpers 96 werden diese, falls im Körperinneren und somit im Bereich des Schafts 18 ein Überdruck besteht, die Schnittflächen 102 zusätzlich gegeneinander gedrückt, um die Schlitze 100 zu verschließen. Damit kann bei laparoskopischen Eingriffen, bei denen im Bauchraum eines Patienten mittels eines Gases ein Überdruck erzeugt wird, um einen Operationssitus freizuhalten, dieser Überdruck gehalten werden, auch dann, wenn Instrumente, oder zum Beispiel in analoger Weise der Obturator 22, mittels des Trokarsystems 10 ins Körperinnere eingeführt werden.

Zu beachten ist ferner, dass das Kreuzschlitzventil 70 erst mittels eines distalen Endes eines Instruments oder beispielsweise der Spitze 236 des Obturators 22 geöffnet werden kann, wenn beim Einführen eines Instruments dessen Schaft, beispielsweise der Schafts 232 des Obturators 22, mittels der Dichtlippe 144 des Dichtelements 74 abgedichtet ist. So ist sichergestellt, dass entweder das Kreuzschlitzventil 70 geschlossen oder eine Abdichtung mittels des Dichtelements 74 relativ zum eingeführten Instrument erfolgt.

Die Trokarhülse 14, der Haltering 72, das Kreuzschlitzventil 70, das Dichtungselement 74, die Schutzvorrichtung 78, die Dichtelementhalterung 76 sowie der Deckel 80 sind jeweils einstückig ausgebildet und vorzugsweise aus einem sterilisierbaren Kunststoffmaterial gespritzt. Der Obturator 22 ist wie beschrieben zweiteilig ausgebildet und kann ebenfalls an einem Kunststoffmaterial durch Spritzgießen hergestellt sein.

Über das Verschlusselement 58 kann bei entsprechender Stellung des Verschlusskolbens 64 ein Gas oder eine Flüssigkeit durch den Schaft 18 ins Innere eines Patientenkörpers eingeleitet oder auch abgeführt werden, selbst dann, wenn ein Instrument, beispielsweise der Obturator 22 in die Trokarhülse 14 eingeführt und ein durch die Trokarhülse 14 definierter Kanal proximalseitig des Anschlussstutzens 54 abgedichtet ist.

## Patentansprüche

1. Chirurgische Dichtelementhalterung (76) zum Halten eines chirurgischen, eine erweiterbare Einführöffnung (176) aufweisenden Dichtelements (74) eines chirurgischen, einen Trokar (14, 22) mit einer Trokarhülse (14) umfassenden Abdichtungssystems (10), wobei die Dichtelementhalterung (76) ein Halterungsdichtelement (140) zum Abdichten der Dichtelementhalterung (76) mit Bezug zu einer inneren Wandfläche (144) der Trokarhülse (14) umfasst und wobei das Halterungsdichtelement (140) in Form eines in radialer Richtung von der Dichtelementhalterung (76) abstehenden Flansches (140) ausgebildet ist, **dadurch gekennzeichnet, dass** der Flansch (140) etwas in distaler Richtung weisend bezogen auf eine quer zu einer Längsachse (12) der Dichtelementhalterung (76) verlaufende Ebene geneigt ist so dass, der Flansch nach Anlegen an eine entsprechende Dichtfläche der Trokarhülse etwas verformt werden kann und so unter Vorspannung gegen die Dichtfläche drücken kann, um eine dauerhafte Abdichtung sicherzustellen.

2. Chirurgische Dichtelementhalterung nach Anspruch 1, **dadurch gekennzeichnet, dass** die Dichtelementhalterung (76) ausgebildet ist zum lösbaren Verbinden mit der Trokarhülse (14).

3. Chirurgische Dichtelementhalterung nach einem der voranstehenden Ansprüche, **dadurch gekennzeichnet, dass** das Halterungsdichtelement (140) einstückig mit der Dichtelementhalterung (76) ausgebildet ist.

4. Chirurgische Dichtelementhalterung nach einem der voranstehenden Ansprüche, **dadurch gekennzeichnet, dass** das Halterungsdichtelement (140) mindestens abschnittsweise elastisch verformbar ist.

5. Chirurgische Dichtelementhalterung nach einem der voranstehenden Ansprüche, **dadurch gekennzeichnet, dass** das Halterungsdichtelement (140) eine weitere Dichtung trägt.

6. Chirurgische Dichtelementhalterung nach Anspruch 5, **dadurch gekennzeichnet, dass** die weitere Dichtung an das Halterungsdichtelement (140) angeformt ist.

7. Chirurgische Dichtelementhalterung nach Anspruch 5 oder 6, **dadurch gekennzeichnet, dass** die weitere Dichtung aus einem Elastomer hergestellt ist.

8. Chirurgisches Abdichtungssystem (10) umfassend eine chirurgische Dichtelementhalterung (76) nach einem der Ansprüche 1 bis 7 und den Trokar (14,22), wobei der Flansch (140) unter Vorspannung gegen die innere Wandfläche (144) drückt beziehungsweise vorgespannt an dieser anliegt.

9. Chirurgisches Abdichtungssystem nach Anspruch 8, **dadurch gekennzeichnet, dass** das Halterungsdichtelement (140) an einer in proximaler oder im Wesentlichen in proximaler Richtung weisenden Ringfläche (36) der Trokarhülse (14) anliegt.

10. Chirurgisches Abdichtungssystem nach Anspruch 9, **dadurch gekennzeichnet, dass** die Ringfläche (36) eine zusätzliche Dichtung trägt.

11. Chirurgisches Abdichtungssystem nach Anspruch 10, **dadurch gekennzeichnet, dass** die zusätzliche Dichtung an die Ringfläche (36) angeformt ist.

12. Chirurgisches Abdichtungssystem nach Anspruch 10 oder 11, **dadurch gekennzeichnet, dass** die zusätzliche Dichtung aus einem Elastomer hergestellt ist.

13. Chirurgisches Abdichtungssystem nach einem der Ansprüche 9 bis 12, **dadurch gekennzeichnet, dass** die Ringfläche (36) durch eine einstufige Durchmesserverjüngung eines Innendurchmesser der Trokarhülse (14) definiert ist.

14. Chirurgisches Abdichtungssystem nach einem der Ansprüche 8 bis 13, **gekennzeichnet durch** eine chirurgische Schutzvorrichtung (78) für das Dichtelement (74), welche Schutzvorrichtung (78) einen am Trokar (14, 22) oder an einem Teil (74) desselben anordenbaren, ringförmig ge-. schlossenen oder im Wesentlichen ringförmig geschlossenen und eine Durchbrechung (192) aufweisenden Grundkörper (190) mit mehreren in Umfangsrichtung angeordneten und parallel oder auf eine Längsachse (12) der Schutzvorrichtung (78) hin weisenden Schutzelementen (200, 202) umfasst, welche Schutzelemente (200, 202) im Wesentlichen in distaler Richtung weisende freie Enden (204, 206) aufweisen, wobei mindestens ein Teil der Schutzelemente (200, 202) an ihren freien Enden (204, 206) oder im Bereich ihrer freien Enden (204, 206) auf einer Außenseite (210) mindestens ein Rückhalteelement (212) zum in Eingriff Bringen mit dem Dichtelement aufweisen.

## Claims

1. Surgical sealing element holder (76) for holding a surgical sealing element (74) of a surgical sealing system (10) comprising a trocar (14, 22) with a trocar sleeve (14), said sealing element (74) having an insertion opening (176) adapted to be widened, wherein the sealing element holder (76) comprises a holder sealing element (140) for sealing the sealing element holder (76) with respect to an inner wall surface (144) of the trocar sleeve (14) and wherein the holder sealing element (140) is designed in the form of a flange (140) projecting from the sealing element holder (76) in a radial direction, **characterized in that** the flange (140) is inclined somewhat in a distal direction with respect to a plane which extends transversely to a longitudinal axis (12) of the sealing element holder (76) so that the flange can be deformed somewhat after abutting on a corresponding sealing surface of the trocar sleeve and can thus press against the sealing surface whilst subject to pretensioning in order to ensure a permanent seal.

2. Surgical sealing element holder according to claim 1, **characterized in that** the sealing element holder (76) is designed for the detachable connection to the trocar sleeve (14).

3. Surgical sealing element holder according to any one of the preceding claims, **characterized in that** the holder sealing element (140) is designed in one piece with the sealing element holder (76).

4. Surgical sealing element holder according to any one of the preceding claims, **characterized in that** the holder sealing element (140) is deformable elastically at least in sections.

5. Surgical sealing element holder according to any one of the preceding claims, **characterized in that** the holder sealing element (140) has an additional seal.

6. Surgical sealing element holder according to claim 5, **characterized in that** the additional seal is integrally formed onto the holder sealing element (140).

7. Surgical sealing element holder according to claim 5 or 6, **characterized in that** the additional seal is produced from an elastomer.

8. Surgical sealing system (10) comprising a surgical sealing element holder (76) according to any one of claims 1 to 7 and the trocar (14, 22), wherein the flange (140) presses against the inner wall (144) or abuts on the inner wall, whilst subject to pretensioning.

9. Surgical sealing system according to claim 8, **characterized in that** the holder sealing element (140) abuts on an annular surface (36) of the trocar sleeve (14) pointing in a proximal direction or essentially in a proximal direction.

10. Surgical sealing system according to claim 9, **characterized in that** the annular surface (36) has an additional seal.

11. Surgical sealing system according to claim 10, **characterized in that** the additional seal is integrally formed onto the annular surface (36).

12. Surgical sealing system according to claim 10 or 11, **characterized in that** the additional seal is produced from an elastomer.

13. Surgical sealing system according to any one of claims 9 to 12, **characterized in that** the annular surface (36) is defined by a one-step narrowing of an inner diameter of the trocar sleeve (14).

14. Surgical sealing system according to any one of claims 8 to 13, **characterized by** a surgical protection device (78) for the sealing element (74), said protection device (78) comprising a base member (190) adapted to be arranged on the trocar (14, 22) or on a part (74) thereof, being closed in a ring shape or closed essentially in a ring shape and having an opening (192) and several protection elements (200, 202) arranged in circumferential direction and pointing parallel or towards a longitudinal axis (12) of the protection device (78), said protection elements (200, 202) having free ends (204, 206) pointing essentially in a distal direction, wherein at least some of the protection elements (200, 202) have at least one retaining element (212) on an outer side (210) at their free ends (204, 206) or in the area of their free ends (204, 206) for engagement with the sealing element.

## Revendications

1. Support d'élément d'étanchéité chirurgical (76) pour maintenir un élément d'étanchéité (74), qui présente une ouverture d'introduction (176) pouvant être évasée, et qui fait partie d'un système d'étanchéité chirurgical (10) comprenant un trocart (14, 22) avec un tube de trocart (14), le support d'élément d'étanchéité (76) comportant un élément d'étanchéité de support (140) pour étancher le support d'élément d'étanchéité (76) par rapport à une surface de paroi intérieure (144) du tube de trocart (14), et l'élément d'étanchéité de support (140) étant configuré sous la forme d'un flasque (140) faisant saillie du support d'élément d'étanchéité (76) dans la direction radiale, **caractérisé en ce que** le flasque (140) est incliné légèrement en direction distale, par rapport à un plan s'étendant transversalement à un axe longitudinal (12) du support d'élément d'étanchéité (76), de sorte que le flasque, après appui contre une surface d'étanchéité correspondante du tube de trocart, peut être légèrement déformé en pouvant ainsi être pressé sous précontrainte contre la surface d'étanchéité, en vue de garantir une étanchéité permanente durable.

2. Support d'élément d'étanchéité chirurgical selon la revendication 1, **caractérisé en ce que** le support d'élément d'étanchéité (76) est configuré pour être relié de manière amovible au tube de trocart (14).

3. Support d'élément d'étanchéité chirurgical selon l'une des revendications précédentes, **caractérisé en ce que** l'élément d'étanchéité de support (140) est réalisé d'un seul tenant avec le support d'élément d'étanchéité (76).

4. Support d'élément d'étanchéité chirurgical selon l'une des revendications précédentes, **caractérisé en ce que** l'élément d'étanchéité de support (140) peut être déformé de manière élastique, au moins par secteurs.

5. Support d'élément d'étanchéité chirurgical selon l'une des revendications précédentes, **caractérisé en ce que** l'élément d'étanchéité de support (140) porte un autre joint d'étanchéité.

6. Support d'élément d'étanchéité chirurgical selon la revendication 5, **caractérisé en ce que** ledit autre joint d'étanchéité est moulé ou formé sur l'élément d'étanchéité de support (140).

7. Support d'élément d'étanchéité chirurgical selon la revendication 5 ou la revendication 6, **caractérisé en ce que** ledit autre joint d'étanchéité est réalisé en un élastomère.

8. Système d'étanchéité chirurgical (10) comprenant un support d'élément d'étanchéité chirurgical (76) selon l'une des revendications 1 à 7, ainsi que le trocart (14, 22), le système étant tel que le flasque (140) soit pressé sous précontrainte contre la surface de paroi intérieure (144) ou s'appuie contre celle-ci sous précontrainte.

9. Système d'étanchéité chirurgical selon la revendication 8, **caractérisé en ce que** l'élément d'étanchéité de support (140) s'appuie sur une surface annulaire (36) du tube de trocart (14), qui est dirigée en direction proximale ou sensiblement en direction proximale.

10. Système d'étanchéité chirurgical selon la revendication 9, **caractérisé en ce que** la surface annulaire (36) porte un joint d'étanchéité supplémentaire.

11. Système d'étanchéité chirurgical selon la revendication 10, **caractérisé en ce que** le joint d'étanchéité supplémentaire est moulé ou formé sur la surface annulaire (36).

12. Système d'étanchéité chirurgical selon la revendication 10 ou la revendication 11, **caractérisé en ce que** le joint d'étanchéité supplémentaire est réalisé en un élastomère.

13. Système d'étanchéité chirurgical selon l'une des revendications 9 à 12, **caractérisé en ce que** la surface annulaire (36) est définie par un rétrécissement de diamètre à un seul étagement, d'un diamètre intérieur du tube de trocart (14).

14. Système d'étanchéité chirurgical selon l'une des revendications 8 à 13, **caractérisé par** un dispositif de protection chirurgical (78) pour l'élément d'étanchéité (74), ledit dispositif de protection (78) comprenant un corps de base (190), qui peut être agencé sur le trocart (14, 22) ou une partie (74) de celui-ci, qui est fermé en forme annuaire ou sensiblement en forme annulaire et présente un passage (192), et qui comporte plusieurs éléments de protection (200, 202) agencés en direction périphérique, parallèles ou dirigés vers un axe longitudinal (12) du dispositif de protection (78), lesdits éléments de protection (200, 202) présentant des extrémités libres (204, 206) dirigées sensiblement en direction distale, au moins une partie des éléments de protection (200, 202) comportant à leurs extrémités libres (204, 206) ou dans la zone de leurs extrémités libres (204, 206), sur un côté extérieur (210), au moins un élément de retenue (212) destiné à être amené en prise avec l'élément d'étanchéité.
